(19) Europäisches Patentamt
European Patent Office
Office européen des brevets

(11) Veröffentlichungsnummer: **0 163 257**
**B1**

# (12) EUROPÄISCHE PATENTSCHRIFT

(45) Veröffentlichungstag der Patentschrift: **13.07.88**

(51) Int. Cl.⁴: **C 08 F 2/50,** G 03 C 1/68

(21) Anmeldenummer: **85106325.5**

(22) Anmeldetag: **23.05.85**

(54) **Photopolymerisierbares Gemisch, das als Photoinitiator ein 1,3,10-Triazaanthracen-4-on enthält.**

(30) Priorität: **01.06.84 DE 3420425**

(43) Veröffentlichungstag der Anmeldung: **04.12.85 Patentblatt 85/49**

(45) Bekanntmachung des Hinweises auf die Patenterteilung: **13.07.88 Patentblatt 88/28**

(84) Benannte Vertragsstaaten: **AT BE CH DE FR GB IT LI NL SE**

(56) Entgegenhaltungen: **EP - A - 0 102 586**

(73) Patentinhaber: **HOECHST AKTIENGESELLSCHAFT, Postfach 80 03 20, D-6230 Frankfurt am Main 80 (DE)**

(72) Erfinder: **Wingen, Rainer, Dr. Dipl.-Chem., Rotkäppchenweg 10, D-6234 Hattersheim/M.3 (DE)**
Erfinder: **Geissler, Ulrich, Dr.Dipl.-Chem., Altenauer Strasse 34, D-6203 Hochheim am Main (DE)**
Erfinder: **Ruckert, Hans, Dr. Dipl.-Chem., Erbsenacker 21, D-6200 Wiesbaden-Naurod (DE)**

**EP 0 163 257 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein photopolymerisierbares Gemisch, das als wesentliche Bestandteile (a) ein polymeres Bindemittel, (b) eine polymerisierbare Verbindung mit mindestens zwei endständigen ethylenisch ungesättigten Gruppen und einem Siedepunkt oberhalb 100 °C und (c) als Photoinitiator ein 1,3,10-Triazaanthracen-4-on enthält.

Photopolymerisierbare Gemische aus den Bestandteilen (a) und (b) und einer mehrkernigen heterocyclischen Verbindung als Photoinitiator sind bekannt.

In der DE-C 20 27 467 (= GB-A 1 354 541) werden als Initiatoren bestimmte Derivate des Acridins und Phenazins beschrieben.

Aus der DE-C 20 39 861 (= US-A 3 765 898) sind ähnliche Gemische bekannt, die als Initiatoren Chinoxalin- oder Chinazolinderivate enthalten.

Alle diese Verbindungen zeigen hervorragende Initiatorwirkung unter Bestrahlung mit aktinischem Licht, insbesondere von Lichtquellen im nahen Ultraviolettbereich. Sie sind jedoch auf Lichtquellen, wie metallhalogeniddotierte Gasentladungslampen, die vermehrt Anteile in sichtbaren Bereich emittieren, nicht optimal abgestimmt. Um diesem Mangel zu begegnen, wurden in der EP-A 102 586 als Photoinitiatoren 1,3-Diaza-9-thia-anthracen-2,4-dione beschrieben, deren Absorptionsbereich und Initiatoraktivität sich mehr in den sichtbaren Spektralbereich erstreckt. Diese Verbindungen lassen sich verhältnismässig leicht herstellen, sie erfordern jedoch eine Umsetzungsstufe mit einem aromatischen Thiol, die infolge der Geruchsbelästigung besondere Schutzmassnahmen erfordert. Ausserdem neigen die Verbindungen aufgrund der Struktur ihres Grundkörpers dazu, in vielen Lösungsmitteln eine begrenzte Löslichkeit aufzuweisen, so dass ihre Anwendbarkeit eingeschränkt ist.

Aufgabe der Erfindung war es, ein photopolymerisierbares Gemisch mit Photoinitiatoren ähnlich hoher Wirksamkeit wie die bekannten, auch im kurzwelligen sichtbaren Spektralbereich vorzuschlagen, die bei ihrer Synthese keine unangenehm riechenden Stufen durchlaufen und die eine bessere Löslichkeit und Verträglichkeit mit anderen Komponenten des Gemischs aufweisen.

Gegenstand der Erfindung ist ein photopolymerisierbares Gemisch, das als wesentliche Bestandteile

(a) ein polymeres Bindemittel,

(b) eine polymerisierbare Verbindung mit mindestens zwei endständigen ethylenisch ungesättigten Gruppen und mit einem Siedepunkt oberhalb 100 °C und

(c) als Photoinitiator eine dreikernige N-heterocyclische Verbindung enthält.

Das erfindungsgemässe Gemisch ist dadurch gekennzeichnet, dass die N-heterocyclische Verbindung der Formel I

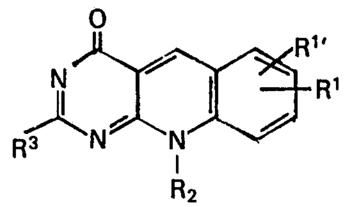

(I)

entspricht, worin

$R^1$ ein Wasserstoff- oder Halogenatom, eine Alkyl-, Alkoxy- oder Dialkylaminogruppe,

$R^{1'}$ ein Wasserstoff- oder Halogenatom oder eine Alkylgruppe und

$R^2$ eine Alkylgruppe oder eine Alkoxycarbonylalkylgruppe ist, wobei

$R^1$ und $R^2$, wenn sie Alkylgruppen bedeuten, miteinander unter Ausbildung eines 5- oder 6-gliedrigen Rings verbunden sein können, und

$R^3$ eine aliphatische oder cycloaliphatische tertiäre Aminogruppe bedeutet.

Die Verbindungen der Formel I sind aus der DE-A 32 33 604 bekannt. Sie werden dort als antimykotisch und antibakteriell wirkende Zwischenprodukte für die Herstellung ähnlich wirkender, in 10-Stellung quaternärer Stickstoffbasen beschrieben. Andere Anwendungsmöglichkeiten werden dort nicht genannt.

Wie erfindungsgemäss gefunden wurde, wirken die Verbindungen bei Bestrahlung im Spektralbereich von etwa 400 bis 450 nm als aktive Radikalstarter für die Photopolymerisation von Vinylverbindungen, auch in Gegenwart von Sauerstoff. Die neuen Photoinitiatoren haben die Eigenschaft, die thermische Polymerisation solcher Verbindungen in Abwesenheit von aktinischer Strahlung nicht zu initiieren. Sie sind somit ideal zur Herstellung lagerfähiger Kopierschichten geeignet.

Wenn in der allgemeinen Formel I $R^1$ und $R^{1'}$ Alkylgruppen sind, haben diese bevorzugt 1 bis 4 Kohlenstoffatome. Wenn $R^1$ eine Alkoxy- oder Dialkylaminogruppe ist, hat auch diese bevorzugt Alkylgruppen mit 1 bis 4, insbesondere 1 oder 2 Kohlenstoffatomen. Als Halogenatome werden Fluor, Chlor und Brom, insbesondere Fluor und Chlor bevorzugt. $R^{1'}$ ist vorzugsweise ein Wasserstoffatom. Von den Verbindungen der Formel I werden diejenigen Isomeren bevorzugt, in denen $R^1$ oder, wenn $R^{1'}$ kein Wasserstoffatom ist, einer der Reste $R^1$ und $R^{1'}$ in der 7-Stellung des Triazaanthracenons steht. Wenn $R^1$ und $R^2$ zu einem Ring verbunden sind, steht $R^1$ im allgemeinen in der 9-Stellung. Die von beiden Resten gebildete Alkylenkette hat dann 2 oder 3 Glieder und kann verzweigt sein. $R^2$ ist vorzugsweise eine Alkylgruppe mit 1 bis 8, besonders bevorzugt 1 bis 4 Kohlenstoffatomen. Wenn $R^2$ eine Alkoxycarbonylalkylgruppe ist, hat diese im allgemeinen eine Alkoxycarbonylgruppe mit 2 bis 4 und eine Alkylgruppe mit 1 bis 4 Kohlenstoffatomen.

Die tertiäre Aminogruppe $R^3$ ist entweder eine Dialkyl- oder Alkylcycloalkylaminogruppe mit 1 bis 8, bevorzugt 1 bis 4 Kohlenstoffatomen in jeder Alkylgruppe und 5 bis 8 Kohlenstoffatomen in

der Cycloalkylgruppe oder eine vollständig oder teilweise hydrierte 5- bis 7-gliedrige N-heterocyclische Gruppe mit 4 bis 10 Kohlenstoffatomen, z.B. eine Pyrrolidino-, Piperidino-, Hexamethylenimino-, Morpholino-, Piperazino-, N-Formylpiperazino-, N-Methyl-piperazino- oder N-Phenylpiperazinogruppe.

Die Photoinitiatoren werden im allgemeinen in einer Menge von 0,01 bis 10, bevorzugt von 0,1 bis 5 Gew.-%, bezogen auf die nichtflüchtigen Bestandteile des photopolymerisierbaren Gemischs, zugesetzt.

Für die Zwecke der Erfindung geeignete photopolymerisierbare Monomere sind bekannt und z.B. in den US-A 2 760 863 und 3 060 023 beschrieben.

Bevorzugte Beispiele sind Acryl- und Methacrylsäureester von zwei- oder mehrwertigen Alkoholen, wie Diglycerindiacrylat, Polyethylenglykoldimethacrylat, Acrylate und Methacrylate von Trimethylolethan, Trimethylolpropan und Pentaerythrit und von mehrwertigen alicyclischen Alkoholen. Mit Vorteil werden auch Umsetzungsprodukte von Diisocyanaten mit Partialestern mehrwertiger Alkohole eingesetzt. Derartige Monomere sind in den DE-A 20 64 079, 23 61 041 und 28 22 190 beschrieben.

Der Mengenanteil der Schicht an Monomeren beträgt im allgemeinen etwa 10 bis 80, vorzugsweise 20 bis 60 Gew.-%.

Als Bindemittel können eine Vielzahl löslicher organischer Polymerisate Einsatz finden. Als Beispiele seien genannt: Polyamide, Polyvinylester, Polyvinylacetale, Polyvinylether, Epoxidharze, Polyacrylsäureester, Polymethacrylsäureester, Polyester, Alkydharze, Polyacrylamid, Polyvinylalkohol, Polyethylenoxid, Polydimethylacrylamid, Polyvinylpyrrolidon, Polyvinylmethylformamid, Polyvinylmethylacetamid sowie Mischpolymerisate der Monomeren, die die aufgezählten Homopolymerisate bilden.

Ferner kommen als Bindemittel Naturstoffe oder umgewandelte Naturstoffe in Betracht, z.B. Gelatine und Celluloseether.

Mit besonderem Vorteil werden Bindemittel verwendet, die wasserunlöslich, aber in wässrig-alkalischen Lösungen löslich oder mindestens quellbar sind, da sich Schichten mit solchen Bindemitteln mit den bevorzugten wässrig-alkalischen Entwicklern entwickeln lassen. Derartige Bindemittel können z.B. die folgenden Gruppen enthalten: $-COOH$, $-PO_3H_2$, $-SO_3H$; $-SO_2NH-$, $-SO_2-NH-SO_2-$ und $-SO_2-NN-CO-$.

Als Beispiele hierfür seien genannt: Maleinatharze, Polymerisate aus N-(p-Tolyl-sulfonyl)-carbaminsäure- β-(methacryloyloxy)-ethylester und Mischpolymerisate dieser und ähnlicher Monomerer mit anderen Monomeren sowie Styrol-Maleinsäureanhydrid-Mischpolymerisate. Alkylmethacrylat-Methacrylsäure-Mischpolymerisate und Mischpolymerisate aus Methacrylsäure, Alkylmethacrylaten und Methylmethacrylat und/oder Styrol, Acrylnitril u. a., wie sie in den DE-A 20 64 080 und 23 63 806 beschrieben sind, werden bevorzugt.

Die Menge des Bindemittels beträgt im allgemeinen 20 bis 90, vorzugsweise 40 bis 80 Gew.-% der Bestandteile der Schicht.

Die photopolymerisierbaren Gemische können je nach geplanter Anwendung und je nach den gewünschten Eigenschaften verschiedenartige Stoffe als Zusätze enthalten. Beispiele sind:

Inhibitoren zur Verhinderung der thermischen Polymerisation der Monomeren,

Wasserstoffdonatoren,

die sensitometrischen Eigenschaften derartiger Schichten modifizierende Stoffe,

Farbstoffe,

gefärbte und ungefärbte Pigmente,

Farbbildner,

Indikatoren,

Weichmacher usw.

Diese Bestandteile sind zweckmässig so auszuwählen, dass sie in dem für den Initiierungsvorgang wichtigen aktinischen Strahlungsbereich möglichst wenig absorbieren.

Als aktinische Strahlung soll im Rahmen dieser Beschreibung jede Strahlung verstanden werden, deren Energie mindestens der des kurzwelligen sichtbaren Lichts entspricht. Geeignet ist langwellige UV-Strahlung, aber auch Elektronen-, Röntgen- und Laserstrahlung.

Das photopolymerisierbare Gemisch kann für die verschiedensten Anwendungen Einsatz finden, beispielsweise zur Herstellung von Sicherheitsglas, von Lacken, die durch Licht oder Korpuskularstrahlen, z.B. Elektronenstrahlen, gehärtet werden, auf dem Dentalgebiet und insbesondere als lichtempfindliches Kopiermaterial auf dem Reproduktionsgebiet.

Die detaillierte Beschreibung der Erfindung beschränkt sich auf dieses Anwendungsgebiet, jedoch ist die Erfindung nicht hierauf beschränkt. Als Anwendungsmöglichkeiten auf diesem Gebiet seien genannt: Kopierschichten für die photomechanische Herstellung von Druckformen für den Hochdruck, den Flachdruck, den Tiefdruck, den Siebdruck, von Reliefkopien, z.B. Herstellung von Texten in Blindenschrift, von Einzelkopien, Gerbbildern, Pigmentbildern usw. Weiter sind die Gemische zur photomechanischen Herstellung von Ätzreservagen, z.B. für die Fertigung von Namensschildern, von kopierten Schaltungen und für das Formteilätzen, anwendbar. Besondere Bedeutung haben die erfindungsgemässen Gemische als Kopierschichten für die photomechanische Herstellung von Flachdruckformen und von Ätzreservagen, insbesondere als vorsensibilisierte Materialien.

Die gewerbliche Verwertung des Gemischs für die genannten Anwendungszwecke kann in der Form einer flüssigen Lösung oder Dispersion, z.B. als Photoresistlösung, erfolgen, die vom Verbraucher selbst auf einen individuellen Träger, z.B. zum Formteilätzen, für die Herstellung kopierter Schaltungen, von Siebdruckschablonen und dgl., aufgebracht wird. Das Gemisch kann auch als feste lichtempfindliche Schicht auf einem geeigneten Träger in Form eines lagerfähig vorbeschichteten lichtempfindlichen Kopiermaterials, z.B. für

die Herstellung von Druckformen, vorliegen. Ebenso ist es für die Herstellung von Trockenresist geeignet.

Es ist im allgemeinen günstig, die Gemische während der Lichtpolymerisation dem Einfluss des Luftsauerstoffes weitgehend zu entziehen. Im Fall der Anwendung des Gemischs in Form dünner Kopierschichten ist es empfehlenswert, einen geeigneten, für Sauerstoff wenig durchlässigen Deckfilm aufzubringen. Dieser kann selbsttragend sein und vor der Entwicklung der Kopierschicht abgezogen werden. Für diesen Zweck sind z.B. Polyesterfilme geeignet. Der Deckfilm kann auch aus einem Material bestehen, das sich in der Entwicklerflüssigkeit löst oder mindestens an den nicht gehärteten Stellen bei der Entwicklung entfernen lässt. Hierfür geeignete Materialien sind z.B. Wachse, Polyvinylalkohol, Polyphosphate, Zucker usw.

Als Schichtträger für mit dem erfindungsgemässen Gemisch hergestellte Kopiermaterialien sind beispielsweise Aluminium, Stahl, Zink, Kupfer und Kunststoff-Folien, z.B. aus Polyethylenterephthalat oder Celluloseacetat, sowie Siebdruckträger, wie Perlongaze, geeignet.

Die Herstellung der lichtempfindlichen Materialien unter Verwendung des erfindungsgemässen Gemischs erfolgt in bekannter Weise.

So kann man dieses in einem Lösungsmittel aufnehmen und die Lösung bzw. Dispersion durch Giessen, Sprühen, Tauchen, Antrag mit Walzen usw. auf den vorgesehenen Träger als Film antragen und anschliessend antrocknen. Dicke Schichten (z.B. von 250 µm und darüber) werden vorteilhaft durch Extrudieren oder Verpressen als selbsttragende Folie hergestellt, welche dann ggf. auf den Träger laminiert wird. Im Falle von Trockenresist werden Lösungen des Gemischs auf transparente Träger aufgebracht und angetrocknet. Die lichtempfindlichen Schichten – Dicke etwa zwischen 10 und 100 µm – werden dann gleichfalls zunächst durch Laminieren zusammen mit dem temporären Träger auf das gewünschte Substrat auflaminiert.

Die Verarbeitung der Kopiermaterialien wird in bekannter Weise vorgenommen. Zur Entwicklung werden sie mit einer geeigneten Entwicklerlösung, bevorzugt einer schwach alkalischen wässrigen Lösung, behandelt, wobei die unbelichteten Anteile der Schicht entfernt werden und die belichteten Bereiche der Kopierschicht auf dem Träger zurückbleiben.

Im folgenden werden Beispiele für das erfindungsgemässe Gemisch angegeben. Dabei wird zunächst die Herstellung einer Anzahl neuer Photoinitiatoren gemäss der Erfindung beschrieben.

In den Herstellungsvorschriften und den nachfolgenden Beispielen stehen Gewichtsteile (Gt) und Volumenteile (Vt) im Verhältnis von g zu ccm. Sofern nicht anders angegeben, verstehen sich Prozent- und Mengenverhältnisse in Gewichtseinheiten.

Die Herstellung der erfindungsgemässen Verbindungen erfolgt wie in Schema 1 angegeben, wobei $R^1$, $R^{1'}$, $R^2$ und $R^3$ die oben genannte Bedeutung haben.

Schema 1

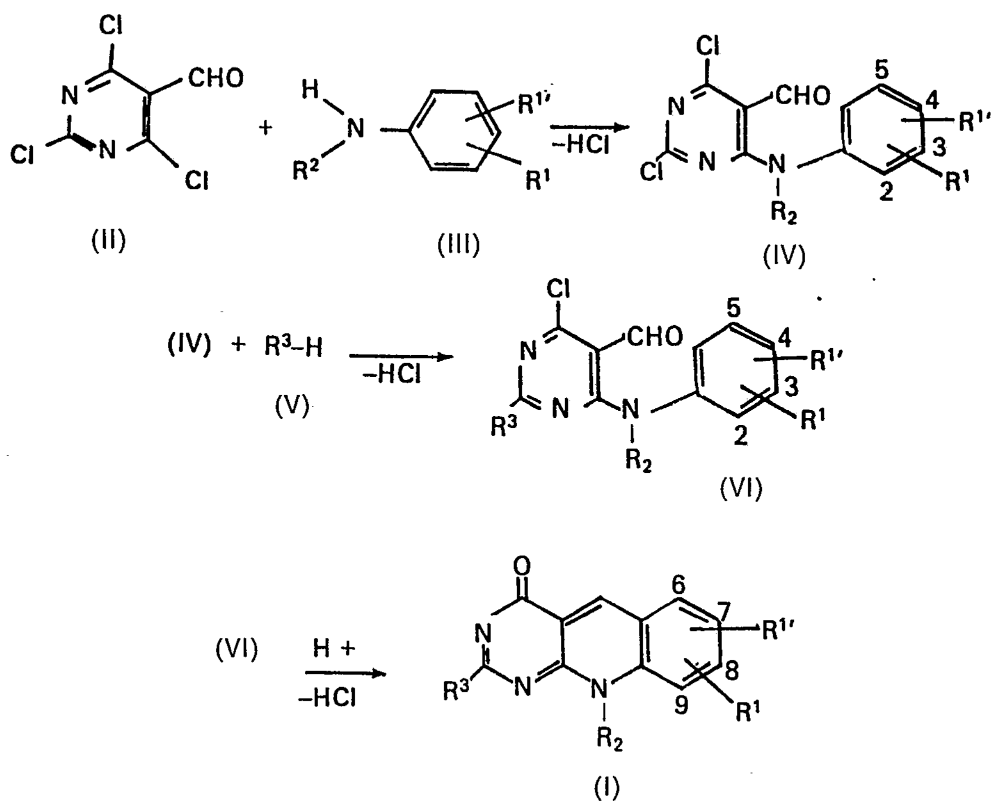


(II) (III) (IV) (V) (VI) (I)

Äquimolare Mengen 2,4,6-Trichlorpyrimidin-5-carbaldehyd der Formel II und sekundäres aromatisches Amin der Formel III werden in einem unter den Reaktionsbedingungen inerten Lösungsmittel wie Benzol, Diethylether, Dioxan, Tetrahydrofuran oder Toluol, bevorzugt Tetrahydrofuran, bei einer Temperatur zwischen 0 und 30 °C, in Gegenwart eines Säurefängers wie tertiäres Amin, Pyridin, Alkali- und Erdalkalicarbonat und -hydrogencarbonat, vorzugsweise Triethylamin, zu einer Verbindung der Formel IV umgesetzt.

Die Umsetzung einer Verbindung der Formel IV mit zweckmässig 1,0 bis 1,1 Äquivalenten eines sekundären Amins der Formel V in einem der zur Herstellung von IV angegebenen Lösungsmittel in Gegenwart der oben beschriebenen Säurefänger bei einer Temperatur zwischen −50 und −20 °C führt zu einer Verbindung der Formel VI, die isoliert werden kann, vorteilhaft jedoch ohne Isolierung weiter umgesetzt wird.

Die Verbindungen der Formel VI werden in konzentrierter Schwefelsäure bis zum Ende der HCl-Entwicklung auf 70–150 °C erhitzt. Nach Hydrolyse können von Nebenreaktionen herrührende Verunreinigungen durch Filtration entfernt werden. Das reine I wird dann aus seiner verdünnt schwefelsauren Lösung durch Neutralisation mit Alkalihydroxid gefällt.

Mit wesentlich längeren Reaktionszeiten kann diese Reaktion auch in Polyphosphorsäure bei 110–160 °C oder in Trifluoressigsäure/Schwefelsäure bei 40–50 °C durchgeführt werden.

Das Herstellungsverfahren wird im folgenden am Beispiel der Verbindung 7 und deren Ausgangsmaterialien näher beschrieben; es ist auf allen Stufen repräsentativ für eine Anzahl analoger Reaktionen, deren Resultate in den Tabellen 1 bis 3 aufgeführt sind.

Verbindung Nr. 7:
7-Chlor-2-diethylamino-10-ethyl-4,10-dihydro-1,3,10-triazaanthracen-4-on

27,3 g (0,074 mol) 4-Chlor-6- (4-chlor-N-ethyl-anilino)- 2-diethylamino-pyrimidin-5-carbaldehyd werden in 550 ml 85 °C warme Schwefelsäure (96%) gegeben. Das Gemisch wird bis zum Ende der HCl-Entwicklung auf 130 °C erhitzt, auf 3 l Eis gegossen, filtriert und mit Natriumhydroxid auf pH 5 gebracht. Der ausgefallene Feststoff wird nach dem Erkalten abgesaugt. Nach Umkristallisation aus 150 ml Ethanol resultieren 15,5 g gelbe Kristalle.

Tabelle 1

| Verbindung Nr. | $R^{1'}$ | $R^1$ | $R^2$ | $R^3$ | Schmp. (°C) | Ausbeute (%) |
|---|---|---|---|---|---|---|
| 1 | H | H | $C_2H_5$ | $-N(C_2H_5)_2$ | 229–231 | 32,9 |
| 2 | H | 7-$CH_3$ | $C_2H_5$ | $-N(C_2H_5)_2$ | 193–195 | 15,8 |
| 3 | H | 7-$CH_3$ | $C_2H_5$ | 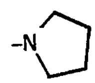 | 295 Z. | 31,5 |
| 4 | 7-$CH_3$ | 8-$CH_3$ | $C_2H_5$ | $-N(C_2H_5)_2$ | 250 Z. | 27,8 |
| 5 | H | (9) $-CH_2CH_2CH_2-$ | | $-N(C_2H_5)_2$ | 215 Z. | 3,2 |
| 6 | H | 7-F | $C_2H_5$ | 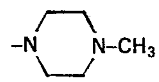 | 217 Z. | 26,4 |
| 7 | H | 7-Cl | $C_2H_5$ | $-N(C_2H_5)_2$ | 241–242 | 63,1 |
| 8 | H | 7-Cl | $C_2H_5$ | $-N(C_4H_9)_2$ | 225–227 | 41,3 |
| 9 | H | 7-Cl | $C_2H_5$ | 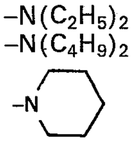 | 305 Z. | 21,4 |
| 10 | H | 7-Cl | $C_2H_5$ | 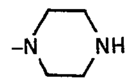 | 250 Z. | 66,5 |
| 11 | H | 7-Cl | $C_2H_5$ | 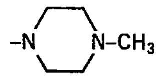 | 240–242 | 49,6 |
| 12 | H | 7-Cl | $C_2H_5$ | 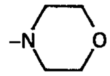 | 295 Z. | 54,0 |

4-Chlor-6- (4-chlor- N-ethyl-anilino)- 2-diethylamino-pyrimidin-5-carbaldehyd
Formel VI: $R^1$=4-Cl, $R^{1'}$=H, $R^2$=$C_2H_5$, $R^3$=$-N(C_2H_5)_2$

Zur Lösung von 40,0 g (0,121 mol) 6-(4-Chlor-N-ethyl-anilino) 2,4-dichlor-pyrimidin-5-carbaldehyd in 400 ml Tetrahydrofuran (THF) wird innerhalb von 5 Stunden bei −30 °C eine Lösung von 9,28 g (0,127 mol) Diethylamin und

12,84 g (0,127 mol) Triethylamin in 80 ml THF getropft. Es wird noch 4 Stunden bei gleicher Temperatur und 2 Stunden bei 20 °C gerührt, das Triethylammoniumchlorid abfiltriert und das Filtrat im Vakuum zur Trockne konzentriert. Nach Verrühren mit 120 ml Diethylether resultieren 27,3 g farblose Kristalle.

Tabelle 2

| $R^{1'}$ | $R^1$ | $R^2$ | $R^3$ | Schmp. (°C) | Ausbeute (%) |
|---|---|---|---|---|---|
| H | H | $C_2H_5$ | $-N(C_2H_5)_2$ | roh weiterverarbeitet | |
| H | 4-$CH_3$ | $C_2H_5$ | 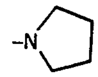 | roh weiterverarbeitet | |
| 3-$CH_3$ | 4-$CH_3$ | $C_2H_5$ | $-N(C_2H_5)_2$ | roh weiterverarbeitet | |
| H | (2) $-CH_2CH_2CH_2$ | | $-N(C_2H_5)_2$ | roh weiterverarbeitet | |
| H | 4-F | $C_2H_5$ | 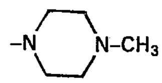 | roh weiterverarbeitet | |
| H | 4-Cl | $C_2H_5$ | $N(C_4H_9)_2$ | roh weiterverarbeitet | |
| H | 4-Cl | $C_2H_5$ | $N(C_2H_5)_2$ | 93–95 | 61,4 |
| H | 4-Cl | $C_2H_5$ | 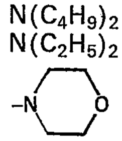 | 142–144 | 62,2 |
| H | 4-Cl | $C_2H_5$ | 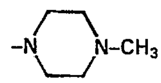 | roh weiterverarbeitet | |
| H | 4-Cl | $C_2H_5$ | 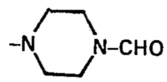 | 182–185 | 41,6 |

6-(4-Chlor-N-ethyl-anilino)- 2,4-dichlor-pyrimidin-5-carbaldehyd
Formel IV: $R^1$=4-Cl, $R^{1'}$=H, $R^2$=$C_2H_5$

Zur Lösung von 21,14 g (0,1 mol) 2,4,6-Trichlorpyrimidin-5-carbaldehyd (II) in 100 ml THF werden nacheinander Lösungen von 15,56 g (0,1 mol) 4-Chlor-N-ethylanilin (III) und 11,13 g (0,11 mol) Triethylamin in jeweils 50 ml THF getropft. Es wird 3 Stunden bei Raumtemperatur nachgerührt, das Triethylammoniumchlorid abfiltriert, das Filtrat im Vakuum zur Trockne gebracht und bei 0 °C mit 150 ml Methanol ausgerührt. Man erhält 23,9 g farblose Kristalle.

Tabelle 3

| $R^{1'}$ | $R^1$ | $R^2$ | Schmp. (°C) | Ausbeute (%) |
|---|---|---|---|---|
| H | H | $CH_3$ | 104–106 | 59,2 |
| H | H | $C_2H_5$ | 117–118 | 39,0 |
| H | 2-$CH_3$ | $CH_3$ | 82– 84 | 33,3 |
| H | 4-$CH_3$ | $C_2H_5$ | 127–130 | 44,5 |
| 3-$CH_3$ | 4-$CH_3$ | $C_2H_5$ | 125–127 | 59,1 |
| H | (2) $-CH_2CH_2-$ | | 163–165 | 40,6 |
| H | (2) $-CH_2CH_2CH_2-$ | | 143–146 | 72,2 |
| H | 2-$OCH_3$ | $C_2H_5$ | 95 Z. | 42,9 |
| H | 4-$OCH_3$ | $C_2H_5$ | 100–103 | 78,5 |
| H | 4-F | $C_2H_5$ | 132–133 | 71,3 |
| 3-Cl | 4-Cl | $C_2H_5$ | 153–156 | 30,1 |
| H | 4-Cl | $C_2H_5$ | 138–139 | 72,3 |

Beispiel 1

Es wurden Beschichtungslösungen hergestellt aus:

4,7 Gt eines Copolymerisats aus 95% Methylmethacrylat und 5% Dimethylaminoethylmethacrylat mit einem mittleren Molekulargewicht $M_w$ von ca. 40 000,

3,3 Gt Trimethylolpropantriacrylat,

0,05 Gt Leukokristallviolett,

0,02 Gt 1,4-Bis- (4-tert.-butoxy-phenylamino)-5,8-dihydroxyanthrachinon

und folgenden Photoinitiatoren

a) 0,1 Gt Verbindung 2
b) 0,1 Gt Verbindung 4
c) 0,1 Gt Verbindung 8
d) 0,03 Gt Verbindung 9 in

19 Gt Butanon.

Die Lösungen wurden auf biaxial verstreckte und thermofixierte Polyethylenterephthalatfolien der Stärke 23 µm so aufgeschleudert, dass nach dem Trocknen bei 100 °C Schichtgewichte von 38 g/m² erhalten wurden.

Die so hergestellten Trockenresistfolien wurden mit einer handelsüblichen Laminiervorrichtung bei 120 °C auf eine mit 35 µm starker Kupferfolie kaschierte Phenoplast-Schichtstoffplatte laminiert und 40 Sekunden mittels einer 5 kW-Metallhalogenidlampe im Abstand von 110 cm zwischen Lampe und Vakuumkopierrahmen belichtet. Als Vorlage diente ein 13-stufiger Belichtungskeil mit Dichteinkrementen von 0,15 und eine Strichvorlage mit Linienbreiten und -abständen bis herab zu 80 µm.

Nach der Belichtung wurde die Polyesterfolie abgezogen und die Schicht mit 1,1,1-Trichlorethan in einem Sprühentwicklungsgerät innerhalb 20 Sekunden entwickelt.

Es wurde die folgende Anzahl vollvernetzter Keilstufen erhalten:

a) = 4; b) =3; c) = 5; d) = 5

Beispiel 2

Auf die in Beispiel 1 genannte Polyesterfolie wurden Lösungen folgender Zusammensetzung so aufgeschleudert, dass nach dem Trocknen ein Schichtgewicht von jeweils 40 g/m$^2$ erhalten wurde:

a) 4,7 Gt des in Beispiel 1 beschriebenen Copolymerisats,
3,3 Gt Trimethylolpropantriacrylat,
0,5 Gt Leukokristallviolett,
0,02 Gt des grünen Anthrachinonfarbstoffs aus Beispiel 1 und
0,05 Gt Verbindung 7 in
19 Gt Butanon
b) Lösung wie a), jedoch Zusatz von
0,075 Gt Ethyl-4-dimethylaminobenzoat.

Die Trockenresistfolien wurden wie in Beispiel 1 beschrieben laminiert, belichtet und entwickelt, wobei für Schicht a) 6 und für Schicht b) 8 (9) vollvernetzte Keilstufen erhalten wurden.

Die entwickelten Platten wurden 30 Sekunden mit Leitungswasser gespült, 30 Sekunden in einer 15%igen Ammoniumperoxydisulfat-Lösung angeätzt, erneut mit Wasser gespült, 30 Sekunden in 10%ige Schwefelsäure getaucht und sodann nacheinander in den folgenden Elektrolytbädern galvanisiert:

1) 45 Minuten in einem Kupferelektrolytbad der Firma Schlötter, Geislingen/Steige, Typ «Glanzkupfer-Bad»
Stromdichte: 2,5 A/dm$^2$
Metallaufbau: ca. 22 µm
Temperatur: Raumtemperatur
2) 15 Minuten in einem Nickelbad Typ «Norma» der Firma Schlötter, Geislingen/Steige
Stromdichte: 3,5 A/dm$^2$
Metallaufbau:10 µm
Temperatur: 50 °C
1) galvanische Verkupferung wie oben
2) 20 Minuten in einem Bleizinnbad LA der Firma Schlötter, Geislingen/Steige
Stromdichte: 1,5 A/dm$^2$
Metallaufbau: 20 µm
Temperatur: Raumtemperatur

Die Platten zeigten keinerlei Unterwanderungen oder Beschädigungen.

Beispiel 3

Die in Beispiel 2 beschriebenen Resistfolien wurden auf einen gereinigten Träger, der aus einem Isolierstoffmaterial mit 35 µm Kupferauflage bestand, laminiert, 40 Sekunden durch eine Leiterbildvorlage belichtet und anschliessend mit 1,1,1-Trichlorethan entwickelt. Das freigelegte Kupfer wurde mit ammoniakalischer Kupferchloridlösung (pH 8,5) bei 48–50 °C weggeätzt. Die Resistschichten zeigen eine hervorragende Resistenz gegenüber dem eingesetzten Ätzmedium.

Beispiel 4

Zur Herstellung einer Trockenresistfolie wurde die folgende Beschichtungslösung eingesetzt:

4,7 Gt eines Copolymerisats aus 98% Methylmethacrylat und 2% Methacrylsäure mit einem mittleren Molekulargewicht $M_w$ von ca. 35 000,
3,3 Gt Trimethylolpropantriacrylat,
0,5 Gt Leukokristallviolett,
0,02 Gt des Anthrachinonfarbstoffs an Beispiel 1,
0,05 Gt Verbindung 7 und
0,125 Gt Ethyl-4-dimethylaminobenzoat in 14 Gt Butanon.

Das Trockenschichtgewicht wurde auf 36 g/m$^2$ eingestellt. Die Verarbeitung erfolgte wie in Beispiel 1 beschrieben. Es wurden 9 vollvernetzte Keilstufen erhalten.

Beispiel 5

Eine Lösung aus
6,0 Gt eines Terpolymerisats aus 78% Butylmethacrylat, 20% Methylmethacrylat und 2% Methacrylsäure mit einem mittleren Molekulargewicht von etwa 30 000,
2,0 Gt Trimethylolpropantriacrylat,
0,05 Gt Leukokristallviolett,
0,02 Gt des Anthrachinonfarbstoffes aus Beispiel 1,
0,05 Gt Verbindung 7 und
0,15 Gt Ethyl-4-dimethylaminobenzoat in
14 Gt Butanon
wurde auf eine Polyesterfolie so aufgeschleudert, dass ein Trockenschichtgewicht von 69 g/m$^2$ erhalten wurde. Die Schicht wurde wie in Beispiel 1 beschrieben verarbeitet. Es wurden 8 (9) vollvernetzte Keilstufen erhalten.

Beispiel 6

Es wurden folgende Beschichtungslösungen hergestellt:

4 Gt eines teilweise mit Alkanol veresterten Styrol-Maleinsäureanhydrid-Mischpolymerisats mit einem mittleren Molekulargewicht von ca. 20 000 und einer Säurezahl um 200,
4 Gt eines Diurethans, hergestellt aus 2 mol Glycerin-dimethacrylat und 1 mol Hexamethylendiisocyanat,
0,14 Gt eines blauen Azofarbstoffs, erhalten durch Kuppeln von 2,4-Dinitro-6-chlor-benzoldiazoniumsalz mit 2-Methoxy-5-acetylamino-N-cyanoethyl-N-hydroxyethylanilin
und folgenden Photoinitiatoren:
a) 0,21 Gt Verbindung 10 oder
b) 0,25 Gt Verbindung 11 in
70 Gt Butanon,
40 Gt Butylacetat und
20 Gt Ethylenglykolmonomethylether.

Die Lösungen wurden auf elektrochemisch aufgerauhtes und anodisiertes Aluminium mit einer Oxidschicht von 2 g/m$^2$, das mit einer wässrigen Lösung von Polyvinylphosphonsäure vorbehandelt war, so aufgeschleudert, dass ein Trok-

kengewicht von 2 g/m$^2$ erhalten wurde. Anschliessend wurde die Platte mit einer Polyvinylalkohol-Deckschicht von 4 g/m$^2$ versehen. Die Druckplatte wurde mittels einer 5 kW-Metallhalogenidlampe unter einem 13-stufigen Halbtonkeil 160 Sekunden belichtet.

Anschliessend wurde mit einem Entwickler folgender Zusammensetzung entwickelt:

3,0 Gt Natriummetasilikat $\times 9\ H_2O$,

0,03 Gt nichtionogenes Netzmittel (Kokosfettalkohol-Polyoxyethylenether mit ca. 8 Oxyethyleneinheiten),

0,003 Gt Antischaummittel und

96,967 Gt vollentsalztes Wasser.

Es wurden bei a) 3 (4) und bei b) 1 (2) vollvernetzte Keilstufen erhalten.

## Patentansprüche

1. Photopolymerisierbares Gemisch, das als wesentliche Bestandteile

(a) ein polymeres Bindemittel,

(b) eine polymerisierbare Verbindung mit mindestens zwei endständigen ethylenisch ungesättigten Gruppen und mit einem Siedepunkt oberhalb 100 °C und

(c) als Photoinitiator eine dreikernige N-heterocyclische Verbindung enthält, dadurch gekennzeichnet, dass die N-heterocyclische Verbindung der Formel I

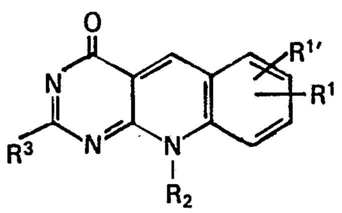

(I)

entspricht, worin

$R^1$ ein Wasserstoff- oder Halogenatom, eine Alkyl-, Alkoxy- oder Dialkylaminogruppe,

$R^{1'}$ ein Wasserstoff- oder Halogenatom oder eine Alkylgruppe und

$R^2$ eine Alkylgruppe oder eine Alkoxycarbonyl-lgruppe ist, wobei

$R^1$ und $R^2$, wenn sie Alkylgruppen bedeuten, miteinander unter Ausbildung eines 5- oder 6-gliedrigen Rings verbunden sein können, und

$R^3$ eine aliphatische oder cycloaliphatische tertiäre Aminogruppe bedeutet.

2. Photopolymerisierbares Gemisch nach Anspruch 1, dadurch gekennzeichnet, dass

$R^3$ eine Dialkylaminogruppe mit 2 bis 16 Kohlenstoffatomen, eine N-Alkyl-cycloalkylaminogruppe mit 1 bis 4 Kohlenstoffatomen im Alkylrest und 5 bis 8 Kohlenstoffatomen im Cycloalkylrest 5- bis 7-gliedrige N-heterocyclische Gruppe mit 4 bis 10 Kohlenstoffatomen ist.

3. Photopolymerisierbares Gemisch nach Anspruch 1, dadurch gekennzeichnet, dass $R^{1'}$ ein Wasserstoffatom ist und $R^1$ in 7-Stellung des Ringsystems der Formel I steht.

4. Photopolymerisierbares Gemisch nach Anspruch 1, dadurch gekennzeichnet, dass $R^1$ in 9-Stellung steht und zusammen mit $R^2$ eine Ethylen- oder Propylengruppe bildet.

5. Photopolymerisierbares Gemisch nach Anspruch 1, dadurch gekennzeichnet, dass die polymerisierbare Verbindung (b) ein Acryl- oder Methacrylsäureester eines mehrwertigen Alkohols ist.

6. Photopolymerisierbares Gemisch nach Anspruch 1, dadurch gekennzeichnet, dass das polymere Bindemittel (a) wasserunlöslich und in verdünnten wässrig-alkalischen Lösungen löslich ist.

## Revendications

1. Mélange photopolymérisable, qui contient comme constituants essentiels:

a) un liant polymère,

b) un composé polymérisable ayant au moins deux groupes à insaturation éthylénique terminaux et ayant un point d'ébullition supérieur à 100 °C et

c) comme photoinitiateur, un composé N-hétérocyclique à trois noyaux, caractérisé en ce que le composé N-hétérocyclique correspond à la formule I

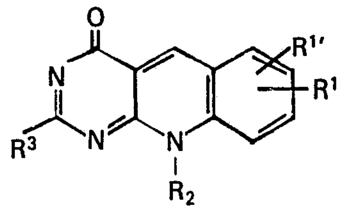

(I)

dans laquelle

$R^1$ est un atome d'hydrogène ou d'halogène, un groupe alkyle, alcoxy ou dialkylamino,

$R^{1'}$ est un atome d'hydrogène ou d'halogène ou un groupe alkyle et

$R^2$ est un groupe alkyle ou un groupe alcoxycarbonylalkyle,

$R^1$ et $R^2$ pouvant, lorsqu'ils représentent des groupes alkyle, être liés l'un à l'autre avec formation d'un cycle à 5 ou 6 chaînons et

$R^3$ représente un groupe amino tertiaire aliphatique ou cycloaliphatique.

2. Mélange photopolymérisable selon la revendication 1, caractérisé en ce que

$R^3$ est un groupe dialkylamino ayant 2 à 16 atomes de carbone, un groupe N-alkyl-cycloalkylamino ayant 1 à 4 atomes de carbone dans le radical alkyle et 5 à 8 atomes de carbone N-hétérocyclique à 5 à 7 chaînons totalement ou partiellement hydrogéné ayant de 4 à 10 atomes de carbone.

3. Mélange photopolymérisable selon la revendication 1, caractérisé en ce que $R^{1'}$ est un atome d'hydrogène et $R^1$ est en position 7 du système cyclique de la formule I.

4. Mélange photopolymérisable selon la revendication 1, caractérisé en ce que $R^1$ est en position 9 et forme avec $R^2$ un groupe éthylène ou propylène.

5. Mélange photopolymérisable selon la revendication 1, caractérisé en ce que le composé polymérisable (b) est un ester acrylique ou méthacrylique d'un polyalcool.

6. Mélange photopolymérisable selon la revendication 1, caractérisé en ce que le liant polymère (a) est insoluble dans l'eau et soluble dans les solutions alcalines aqueuses diluées.

**Claims**

1. Photopolymerizable composition comprising as the essential components:

a) a polymeric binder,

b) a polymerizable compound having at least two terminal, ethylenically unsaturated groups and a boiling point above 100 °C, and

c) a trinuclear N-heterocyclic compound as a photoinitiator, wherein the N-heterocyclic compound corresponds to Formula I

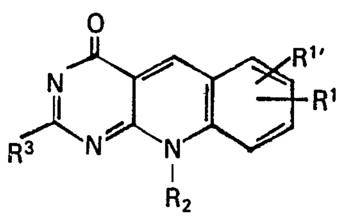 (I)

wherein

$R^1$ is a hydrogen or halogen atom, an alkyl group, alkoxy group, or dialkylamino group,

$R^{1'}$ is a hydrogen or halogen atom or an alkyl group, and

$R^2$ is an alkyl group or an alkoxycarbonylalkyl group,

$R^1$ and $R^2$ may be linked together to form a five or sixmembered ring, if they stand for alkyl groups, and

$R^3$ denotes an aliphatic or cycloaliphatic tertiary amino group.

2. A photopolymerizable composition as claimed in claim 1, wherein

$R^3$ is a dialkylamino group having from 2 to 16 carbon atoms, an N-alkyl-cycloalkylamino group having from 1 to 4 carbon atoms in the alkyl radical and from 5 to 8 carbon atoms in the cycloalkyl radical, or a completely or partially hydrogenated N-heterocyclic group formed of 5 to 7 ring members and having from 4 to 10 carbon atoms.

3. A photopolymerizable composition as claimed in claim 1, wherein $R^{1'}$ is a hydrogen atom and $R^1$ is in the 7-position of the ring system of Formula I.

4. A photopolymerizable composition as claimed in claim 1, wherein $R^1$ is in the 9-position and, together with $R^2$, forms an ethylene group or a propylene group.

5. A photopolymerizable composition as claimed in claim 1, wherein the polymerizable compound (b) comprises an acrylic or methacrylic acid ester of a polyhydric alcohol.

6. A photopolymerizable composition as claimed in claim 1, wherein the polymeric binder (a) is insoluble in water and soluble in dilute aqueous-alkaline solutions.